# EUROPÄISCHE PATENTSCHRIFT

(11) **EP 4 072 519 B1**
(45) Veröffentlichungstag und Bekanntmachung des Hinweises auf die Patenterteilung: **06.08.2025**
(21) Anmeldenummer: 20823745.3
(22) Anmeldetag: 02.12.2020
(51) Int. Cl.: A23P 10/28, A23P 20/20, A61K 9/00, A61K 9/10, A61K 9/16, A61K 9/20, A61K 9/70, B33Y 10/00, A23L 33/10, A23P 20/25, A23P 30/20, B33Y 80/00, B29C 64/118

(54) **SPOTDRUCKVERFAHREN UND -VORRICHTUNG ZUR ADDITIVEN HERSTELLUNG WIRKSTOFFHALTIGER DARREICHUNGSFORMEN**
SPOT PRINTING PROCEDURE AND APPARATUS FOR THE ADDITIVE MANUFACTURING OF DRUG-CONTAINING PHARMACEUTICAL FORMS
PROCÉDÉ D'IMPRESSION GOUTTE À GOUTTE ET APPAREIL POUR LA PRODUCTION ADDITIVE DE FORMES PHARMACEUTIQUES COMPRENANT UN PRINCIPE ACTIF

(30) Priorität: 09.12.2019 EP 19214624
(43) Veröffentlichungstag der Anmeldung: 19.10.2022
(73) Patentinhaber: DiHeSys Digital Health Systems GmbH, 73529 Schwäbisch Gmünd (DE)
(72) Erfinder: DACHTLER, Markus, 89079 Ulm (DE); HUBER, Gerald, 73655 Plüderhausen (DE)
(74) Vertreter: Habermann, Hruschka & Schnabel
(86) Internationale Anmeldenummer: PCT/EP2020/084336
(87) Internationale Veröffentlichungsnummer: WO 2021/115887

(56) Entgegenhaltungen:
- WO-A1-2017/140239
- WO-A1-2017/158172
- WO-A1-2018/151725
- NORMAN JAMES ET AL: "A new chapter in pharmaceutical manufacturing: 3D-printed drug products", ADVANCED DRUG DELIVERY REVIEWS, ELSEVIER, AMSTERDAM , NL, vol. 108, 18 March 2016 (2016-03-18), pages 39 - 50, XP029899913, ISSN: 0169-409X, DOI: 10.1016/J.ADDR.2016.03.001

## Beschreibung

Die vorliegende Erfindung betrifft ein Verfahren und eine Vorrichtung zur Herstellung von festen, mindestens halbfesten Darreichungsformen pharmazeutischer Wirkstoffe, nutrazeutischer Wirkstoffe und/oder von Nahrungsergänzungsmittelwirkstoffen, bei der die Darreichungsform in additiver Weise durch Aufbringen einer Aufbausubstanz in Form von Spots erstellt wird.

Aus der WO 2016/038356 A1 ist ein additives 3D-Druckverfahren zur Herstellung pharmazeutischer Darreichungsformen mittels Filament-Fusionsfabrikation (FFF) bekannt. WO 2017/140239, WO 2017/158172, WO 2018/151725 and NORMAN JAMES ET AL:, ADVANCED DRUG DELIVERY REVIEWS, Bd. 108, 18. März 2016 (2016-03-18), Seiten 39-50 beschäftigen sich zudem mit pharmazeutischen Darreichungsformen, die durch verschiedene Techniken eines 3D-Druck-Verfahrens hergestellt werden.

Aufgabe der vorliegenden Erfindung ist die die Bereitstellung eines Verfahrens und einer Vorrichtung zur Herstellung wirkstoffhaltiger Objekte, insbesondere wirkstoffhaltiger Darreichungsformen, die einen besonders leichten und materialsparenden Aufbau aufweisen.

Die vorstehende Aufgabe wird durch die Ausführungsformen der vorliegenden Erfindung, wie sie in den Ansprüchen und der vorliegenden Beschreibung offenbart werden, gelöst.

Insbesondere stellt die vorliegende Erfindung ein Verfahren zur Herstellung einer mindestens halbfesten Darreichungsform bereit, das die folgenden Schritte umfasst:
(i) Bereitstellen eines mindestens zum 3D-Druck der festen Darreichungsform befähigten Druckers, der
   eine Aufbauplattform, auf der die Darreichungsform gedruckt wird,
   einen zum Auftragen einer Anordnung von Spots einer Aufbausubstanz für die Darreichungsform auf der Aufbauplattform ausgelegten Druckkopf, wobei die Aufbausubstanz im Druckustand, vorzugsweise durch Erwärmung, fließfähig ist und durch Verfestigung, vorzugsweise Abkühlung, mindestens halbfest wird,
(ii) Aufbringen einer Anordnung von Spots der Aufbausubstanz auf der Aufbauplattform, wobei sich die Spots überlappen oder berühren oder nicht berühren können;
(iii) mindestens Halbverfestigen, vorzugsweise Verfestigen, der im Schritt (ii) aufgebrachten Spots der Aufbausubstanz;
(iv) Aufbringen einer weiteren Anordnung von Spots auf die vorherige Anordnung von Spots derart, dass sich die Spots der weiteren Anordnung mit den Spots der vorherigen Anordnung mindestens teilweise überlappen; und
(v) Wiederholgen der Schritte (ii) bis (iv) bis die Darreichungsform gebildet ist,
wobei die Aufbausubstanz mindestens einen pharmazeutischen Wirkstoff und/oder mindestens einen nutrazeutischen Wirkstoff und/oder mindestens einen Nahrungsergänzungsmittelwirkstoff enthält;

Ein "Spot" im Sinne der Erfindung ist ein im Wesentlichen rundes, an sich dreidimensionales Gebilde, dass aus dem Auftreffen einer Volumeneinheit der beim Aufbringen der Aufbausubstanz entsteht, die aus einem Druckkopf der Druckvorrichtung in flüssiger, mindestens jedoch fließfähiger Form üblicherweise in Form eines Tropfens, (angenäherten) Rotationsellipsoids oder einer (angenäherten) Kugel ausgegeben wird und auf der Aufbauplattform (im erfindungsgemäßen Schritt (ii)) oder, jedenfalls teilweise, auf bereits zuvor abgeschiedenen Spots (im Schritt (iii) bzw. den Schritten (iii)) abgeschieden wird.

Erfindungsgemäß enthält die Aufbausubstanzt mindestens einen Wirkstoff, der aus pharmazeutischen, nutrazeutischen und Nahrungsergänzungsmittelwirkstoffen ausgewählt sein kann. Nachstehend werden diese Wirkstoffe zusammenfassend auch als "aktive Komponente(n)" oder auch als "Wirkstoff(e)" bezeichnet.

Die aktiven Komponenten sind im Wesentlichen aus sämtlichen verfügbaren und mittels halbfester oder fester Darreichungsformen verabreichbaren pharmazeutischen, nutrazeutischen und Nahrungsergänzungsmittelwirkstoffen auswählbar, die wiederum auch untereinander kombinierbar sind, soweit dies für die gewünschte Anwendung verträglich ist. Bevorzugte Darreichungsformen der Erfindung werden mit Hilfe von Aufbausubstanzen hergestellt, die mindestens einen pharmazeutischen Wirkstoff enthalten. Bevorzugte Ausführungsformen sind synergistisch wirkende Kombinationen von 2 oder mehr pharmazeutischen Wirkstoffen, die in einer einzelnen Aufbausubstanz vorliegen können. In einer anderen Ausführungsform können unterschiedliche Wirkstoffe in unterschiedlichen

Aufbausubstanzen vorliegen, welche die aufgebrachten Spots bilden. So kann erfindungsgemäß ein Wirkstoff in einer Aufbausubstanz vorliegen, aus der eine erste Gruppe von Spots gebildet werden und ein (oder mehrere) anderer Wirkstoff(e) in einer anderen Gruppe von Spots enthalten sein (d.h. es liegen mindestens zwei Aufbausubstanzen vor, welche den bzw. die jeweiligen Wirkstoffe enthalten). bevorzugt sind die verschiedenen Wirkstoffe in unterschiedlichen Aufbausubstanzen enthalten. Es ist dabei möglich, dass die jeweiligen Aufbausubstanzen mit Ausnahme des oder der Wirkstoffe ansonsten gleich sein oder unterschiedlich ein können, z.B. um in Abhängigkeit des jeweiligen Wirkstoffs auf diesen zugeschnittene Eigenschaften wie bspw. pH-Wert, Löslichkeit, Konsistenz, lonenmilieu, Partikelgröße, Farbe usw. bereitzustellen. In bestimmten Ausführungsformen der Erfindung werden Kombinationen von 2 oder mehr pharmazeutischen Wirkstoffen bereitgestellt, bei dem z.B. ein für eine bestimmte Indikation vorgesehener pharmazeutischer Wirkstoff in einer Aufbausubstanz enthalten ist und ein weiterer pharmazeutischer Wirkstoff in derselben oder einer anderen Aufbausubstanz vorliegt wie z.B. eine von dem ersten Wirkstoff jedenfalls potentiell hervorgerufene Nebenwirkung mindestens reduziert, bestenfalls unterdrückt, was eine besonders bevorzugte Ausführungsform der Erfindung darstellt.

Beispiele von erfindungsgemäß kombinierbaren pharmazeutischen Wirkstoffen sind Agonisten/Antagonisten zur Reduktion des Suchtpotentials bei Schmerzmitteln (z.B. Tilidin und Naloxon), Kombinationspräparate zur Behandlung von z.B. Bluthochdruck (bevorzugt Kombinationen von ACE-Hemmer und/oder Calciumkanalblocker und/oder Betablocker), Allergien (z.B. Antihistaminika und Calcium), Diarrhöen (z.B. Mineralstoffe und Loperamid), Schlaganfälle/Infarktprophylaxe (Blutverdünner und/oder Blutdrucksenker), Parkinson (Levodopa und Benserazide), Hypercholesterinämie (z.B. Statine und/oder Fischöle), Mangelerscheinungen (z.B. Vitamine mit Mineralstoffen in Kombination) und Analgetika (z.B. ein Analgetikum und Coffein) etc.

In bevorzugten Ausführungsformen der Erfindung befinden sich Spots mit gleichem Wirkstoff oder gleicher Wirkstoffkombination oder gleicher Wirkstoffkonzentration in einem gemeinsamen Abschnitt der Darreichungsform, so dass die Spots der gleichen Gruppe an mindestens einer Seite zumindest teilweise aneinander angrenzen. Bevorzugt bilden also die Spots mit gleichem Wirkstoff oder mit gleicher Wirkstoffkombination oder mit gleicher Wirkstoffkonzentration jeweils mindestens einen gemeinsamen Abschnitt wie (z.B. mindestens eine gemeinsamen Schicht oder mindestens einen zusammenhängenden Teil mindestens einer Schicht, wobei diese horizontal oder vertikal, bezogen auf die längste Dimension der Darreichungsform, ausgerichtet sein können. Spots mit jeweils gleichem Wirkstoff, gleicher Wirkstoffkombination oder gleicher Wirkstoffkonzentration können in anderen Ausführungsformen auch in mehreren Abschnitten (also z.B. 2 oder mehr Schichten und/oder Teilschichten) zusammengefasst sein. Derartige Abschnitte können auch unterschiedliche Wirkstofffreigabeeigenschaften aufweisen wie z.B. unterschiedliche pH-Bedingungen, Löslichkeit, Magensaftresistenz, sonstige Löslichkeitsverhalten (z.B. in dem Spots bestimmter Abschnitte oder eines bestimmten Abschnitts eine Burst-Release-Substanz enthalten, wobei ein Verfahren zur Bildung einer Burst-Release-Ausführungsform bevorzugt derart ausgebildet ist, dass die Spots der Burst-Release-Abschnitte derart aufgebracht werden, dass die Burst-Release-Abschnitte die Spot-Abschnitte ohne Burst-Release-Mittel in der Aufbausubstanz umhüllen, also mindestens eine, vorzugsweise mehrere Schichten von Spots mit Burst-Release-Substanz(en) in der oder den Aufbausubstanzen in jeder Dimension der Darreichungsform die Abschnitte der Darreichungsform ohne Burst-Release-Funktion umgeben).

Vorzugsweise weist der Drucker mindestens einen Druckkopf auf, der mit einem Reservoir mit der Aufbausubstanz in Verbindung steht, so dass der mindestens eine Druckkopf zur Entnahme einer Menge der Aufbausubstanz zum Aufbringen der Aufbausubstanz in den Schritten (ii) bis (v) befähigt ist. Das Reservoir kann dabei in unterschiedlicher Weise in Abhängigkeit von der Art und Konsistenz der Aufbausubstanz ausgestaltet sein. So kann das Reservoir im Falle von flüssigen Aufbausubstanzen ein Flüssigkeitsbehälter sein, der mit dem Druckkopf über eine Leitung für die Aufbausubstanz in Verbindung steht, durch welche diese zum Druckkopf transportiert, üblicherweise gepumpt wird. In einer anderen Ausführungsform kann die Aufbausubstanz in dem Reservoir in fester oder halbfester Form, bspw. als Pulver oder Granulat vorliegen, wobei ein Transportmechanismus die feste oder halbfeste Aufbausubstanz den Druckkopf zuführt. In dieser Ausführungsform weist der Druckkopf typischerweise eine Heiz- oder Schmelzeinrichtung auf, welche die Aufbausubstanz in eine mindestens fließfähige, in bevorzugten Ausführungsformen flüssige Form überführt, welche dann von dem Druckkopf, durch eine üblicherweise vorhandene Ausgabeeinrichtung als eine auf der Aufbauplattform einen Spot bildende Volumeneinheit auf die Aufbauplattform ausgegeben, also gedruckt wird.

In einer anderen Ausführungsform kann die Aufbausubstanz auch in Form eines festen oder jedenfalls halbfesten Filaments vorliegen, wobei das Filament bspw. in einem Zuführungsgang oder einer Zuführungsröhre vorliegt, welche das Reservoir bilden. Dabei können diese Reservoirformen unterschiedlich ausgebildet sein, wobei bei vollständig festen Filamentaufbausubstanzen meist lineare Ausführungsformen vorgesehen sind. Bevorzugte Filamentaufbausubstanzen sind meist längliche, zylinderförmige Gebilde, die typischerweise mehr oder weniger elastisch sind und daher auch in gebogenen wie beispielsweise spiralförmigen Reservoirspulen aufgenommen werden können und bspw. durch einen Schiebe- oder Drückmechanismus dem Druckkopf zugeführt werden können. Sollte die Elastizität nicht ausreichen um das Filament spiralförmig in Reservoirspulen zuzuführen, dann kann das Filament auch in kurzen, geraden Filamentstäben aus einem Reservoirmagazin zugeführt werden.

Im Falle von flüssigen Aufbausubstanzen kann der Druckkopf piezoelektrisch betriebene Einrichtungen zur Ausgabe der Volumeneinheit umfassen, so dass die Aufbausubstanz wie bei einem Tintenstrahldrucker ausgegeben wird. Eine derartige Ausführungsform kann auch als 2D-Druckvorgnang, wie unten genauer ausgeführt, ausgestaltet sein. Bei einem 2D-Druckvorgang wird eine Flüssigkeit (Beispiele sind unten genannt) üblicherweise über bekannte Techniken wie piezoelektrische Ausgabeeinrichtungen bspw. mindestens auf einem Abschnitt der Oberfläche einer durch die obigen Schritte im 3D-Druck erstellten Darreichungsform bspw. in einem zusätzlichen Schritt (vi) aufgebracht, wobei die Flüssigkeit üblicherweise getrocknet oder in anderer Weise auf dem mindestens einen Abschnitt der Oberfläche fixiert wird (z.B. chemisch und/oder durch Lichteinwirkung, was meist durch eine Lasereinrichtung bewirkt wird). Selbstverständlich ist es auch möglich, eine oder mehrere 2D-gedruckte Schichten innerhalb einer Darreichungsform aufzubringen und nach dem Aufbringen einer 2D-Schicht mit den 3D-Druck-Schritten fortzufahren (wobei selbstverständlich eine abschließende 2D-Druckschicht folgen kann).

In einer weiteren bevorzugten Ausführungsform weist der Drucker mehr als einen Druckkopf auf, wobei 2 bis 10 Druckköpfe besonders bevorzugt sind. Ausführungsformen der Erfindung mit mehreren Druckköpfen können unterschiedlichen Funktionen dienen: In einer Ausführungsform ist es vorgesehen, dass mit mehr als einem Druckkopf an einer Einheit der Darreichungsform gedruckt wird, z.B um unterschiedliche Aufbausubstanzen mit den wie vorstehend dargelegten unterschiedlichen Eigenschaften zu verdrucken. Es können bei einer Ausführungsform mit mehreren Druckern auch 3-D- und 2D-Druckköpfe vorgehen werden, wobei es auch möglich ist, wie nachstehend erläutert, dass erfindungsgmäß verwendbare Druckköpfe sowohl zum 3-D-Druck als auch zum 2D-Druck ausgelegt sind. Es ist weiterhin erfindungsgemäß vorgesehen, mehr als einen Druckkopf zu verwenden, um mehrere Darreichungsformen gleichzeitig zu drucken. Es ist erfindungsgemäß selbstverständlich auch möglich, mehrere verschiedene Darreichungsformen gleichzeitig zu drucken, d.h. es werden sozusagen Druckkopfsets gebildet oder jedenfalls als Sets angesprochen, die gleichzeitig mit verschiedenen Aufbausubstanzen an mehreren Darreichungsformen drucken, wobei wiederum die gleichzeitig gedruckten Darreichungsformen in ihrem Aufbau gleich oder unterschiedlich sein können. Die Anzahl der Druckköpfe kann daher auch deutlich über 10 Druckkopfe hinausgehen, um die Anzahl der gedruckten Darreichungsformen entsprechend erhöhen zu können. Bei der Ausführungsform des Verfahrens mit mehr als einem Druckkopf ist es weiterhin bevorzugt, dass jeder der Druckköpfe mit einem Reservoir mit der Aufbausubstanz in Verbindung steht, so dass der jeweilige Druckkopf zur Entnahme einer Menge der Aufbausubstanz zum Aufbringen der Aufbausubstanz in den Schritten (ii) bis (v) befähigt ist. Dabei können die vorstehenden Reservoirausbildungen unabhängig voneinander bei den Druckköpfen gleich oder verschieden sein.

Das Erzeugen der Spots erfolgt also erfindungsgemäß bevorzugt durch Aufbringen einer Volumeneinheit der Aufbausubstanz, wobei eine Volumeneinheit vorzugsweise ein Volumen von 20 pl bis 30 µl aufweist. Bei Bedarf können natürlich auch mehrere Volumeneinheiten hintereinander aufgebracht werden.

Gemäß einer weiteren bevorzugten Ausführungsform ist der Druckkopf oder, im Falle von mehreren Druckköpfen, mindestens einer der Druckköpfe sowohl zum 2D-Druck als auch zum 3D-Druck ausgelegt ist. In einer anderen Ausführungsform der Erfindung weist die Vorrichtung mindestens einen oder mehrere 3D-Druckkopfe und bei Bedarf einen 2D-Druckkopf auf. 3D-Druckköpfe sind zum Aufbringen von halbfesten und geschmolzenen Aufbausubstanzen ausgelegt, während 2D-Druckköpfe zur Aufbringung von bereits ohne Erhitzung im Druckkopf flüssigen Aufbausubstanzen wie bspw. Tinten oder Wirkstofflösungen, Wirkstoffemulsionen und Wirkstoffsuspensionen ausgelegt sind.

In einer bevorzugten Ausführungsform werden die Spots derart aufgebracht, dass sich die im Schritt (ii) und den weiteren Schritten (ii) (gemäß Schritt (v) des erfindungsgemäßen Verfahrens) mit den im vorherigen Schritt (Schritt (ii) bzw. jeder Schritt (ii) der weiteren Aufbauschritte gem. Schritt (v)) aufgebrauchten Spots überlappt. Diese Ausführungsform ergibt somit eine Darreichungsform, die im Sinne einer Backsteinanordnung der aufgebrachten Spots eine besonders stabile Anordnung bilden kann, falls die Spots einer Schicht eine vollständige Überlappung mit der vorhergehenden Schicht aufweisen. Andererseits wird durch Aufbringen einer Schicht von Spots, die mit der vorhergehenden Schicht teilweise überlappen, eine besonders leichte Anordnung mit Zwischenräumen bereitgestellt, wodurch die durch Erzeugung einer größeren dem umgebenden Milieu ausgesetzten Oberfläche der Darreichungsform deren Löslichkeit oder Degradationsgeschwindigkeit der im umgebenden Milieu, insbesondere im Verdauungstrakt des die Darreichungsform einnehmenden Subjekts, bspw. einen humanen Patienten, gesteuert werden kann,

Wie vorstehend beschrieben, liegt die Aufbausubstanz in bevorzugten Ausführungsformen der Erfindung als Filament vor und der Druckkopf ist, im Falle des 3D-Druckes, zum Abschmelzen einer Menge des Filaments ausgelegt, vorzugsweise indem der Druckkopf, wie vorstehend beschrieben, eine Heizeinrichtung umfasst, um die Anordnung von Spots der Aufbausubstanz auf der Aufbauplattform im Schritt (iii) sowie die weitere(n) Anordnung(en) von Spots auf der vorherigen Anordnung von Spots im Schritt (iv) aufzubringen.

Die Verfestigung (mindestens in einen halbfesten Zustand der gedruckten Aufbausubstanz sowie die Bindung zwischen den einzelnen aufgebrachten Spots im Schritt (iii) kann auf unterschiedliche Weise erfolgen. In einer Ausführungsform kann bspw. bei Verwendung eines schmelzbaren Materials die Bindung zwischen solchen Spots durch die Verfestigung nach der Aufbringung auf die Trägerstruktur erfolgen, wobei diese durch verschiedene Mechanismen wie simple Abkühlung und/oder chemisch durch bekannte Substanzen durchgeführt werden kann. In einer anderen Ausführungsform kann der Aufbausubstanz z.B. einer Dispersion oder einer Lösung ein geeignetes Bindemittel zugesetzt werden, dass nach dem Aufbringen des Spots dieses zum Aushärten bringt, wobei die Aushärtung durch das Bindemittel bspw. durch Wärme erfolgen kann, die durch eine geeignete Wärmequelle in der Druckvorrichtung zugeführt werden kann wie bspw. einer Lichtquelle, vorzugsweise einer Laser-Einrichtung. Das Aushärten durch das Bindemittel kann auch chemisch durch entsprechende Startermoleküle und/oder Licht einer geeigneten Wellenlänge erfolgen, wobei wiederum letzteres bevorzugt mit Hilfe einer Lasereinrichtung emittiert wird. In einer weiteren Ausführungsform kann die Aufbausubstanz ein oder mehrere Ausgangsverbindungen, typischerweise Monomere, eines oder mehrerer Polymere enthalten, und nach dem Aufbringen des/der Spots wird durch geeignete Mittel wie bspw. wiederum Licht, Wärme oder andere Polymerisationsstarter eine Polymerisation in Gang gesetzt, welche den jeweils aufgebrachten Spot aushärtet und mit benachbarten Spots verbindet bzw. verklebt.

Geeignete bei der Drucktemperatur fließfähige Träger-Materialien, in welchen der oder die Wirkstoff(e) vorliegt/vorliegen, sind z.B. im Allgemeinen für eine Schmelzextrusion (engl. hot melt extrusion, HME) verwendbare Träger wie niedrig schmelzende Wachse und Polymere. Das HME-Gemisch bzw. allgemein das Aufbausubstanzgemisch kann neben dem niedrig schmelzenden Träger weitere Prozessmittel und Hilfsstoffe wie Bindemittel, Weichmacher, Antioxidantien, Duftstoffe, Süßstoffe oder ähnliches enthalten. Geeignete HME-Träger und Weichmacher sind dem Fachmann bekannt und z.B. in Crowley et al. (2007) Drug Development and Industrial Pharmacy, 33, 909-926, offenbart (Träger: Seiten 917 bis 919, insbesondere Tabelle 1; Weichmacher: Seiten 917 und 920, insbesondere Tabelle 2), wobei in der vorliegenden Beschreibung *expressis verbis* auf die genannten Passagen Bezug genommen wird.

Das vorliegende additive 3D-Druckverfahren bzw. das vorliegende kombinierte additive 3D- und 2D-Druckverfahren wird bevorzugt computergestützt durchgeführt. So wird typischerweise im einem dem Schritt (i) vorgelagerten Schritt ein berechnetes zwei- oder dreidimensionales Bild des zu druckenden Objekts z.B. mit Hilfe eines gängigen CAD-Programms erstellt. Die computergenerierte Widergabe des zu druckenden Objekts kann auch durch scannen eines bereits existierenden Objekts, z.B. einer pharmazeutischen Darreichungsform oder eines Medizinprodukts, erfolgen. Das computergenerierte Modellbild wird dann bei dem vorliegenden Verfahren in die gewünschten Spots unterteilt, wobei die Auflösung des realen Objekts umso größer wird, je kleiner die zur Erstellung der Spots aufgebrachten Volumeneinheiten der Aufbausubstanz sind. Jedem einzelnen Spot kann bspw. ein Wirkstoff, eine Trägersubstanz bzw. Trägerzusammensetzungen und/oder weitere Hilfsmittel wie Farbsubstanzen, pH-Wert abhängig lösliche Substanzen, Temperaturabhängig schmelzende Substanzen, und andere ggf. benötigte Materialien sowie deren Menge (Konzentration in der zur Aufbringung des Spots abgeschiedenen Volumeneinheit) zugeordnet und schließlich gedruckt werden. Hinsichtlich geeigneter Bestandteile für übliche Druckvorrichtungen wird z.B. auf US 2017/03,68755 A1 und US 6,070,107 B2 verwiesen.

Mit dem Verfahren druckbare wirkstoffhaltige Objekte sind insbesondere halbfeste oder feste pharmazeutische Darreichungsformen, wie z.B. Tabletten, Kapseln, Implantate, Pflaster, Zäpfchen, Pellets, gedruckte Granulate oder orale bzw. topische Dünnfilme. Mit Hilfe des erfindungsgemäßen Verfahrens herstellbare Tabletten sind vielfältig und schließen zum Beispiel Oblongtabletten, Lutschtabletten, Implantattabletten, Multiple-Applikation-Tabletten, Disperstabletten, Retardtabletten, Vaginaltabletten, Augentabletten, Manteltabletten, Matrixtabletten, Kautabletten, Filmtabletten, Modified-Release-Tabletten, Lacktabletten und margensaftresistente Tabletten ein.

Weitere besonders geeignete Objekte sind Medizinprodukte wie z.B. wirkstoffhaltige topische Arzneiformen, Kontaktlinsen, Pflaster, welche vorzugsweise den oder die Wirkstoffe, vorzugsweise zur lokalen Anwendung, freisetzen. Das erfindungsgemäße Verfahren wird in einer Ausführungsform unter sterilen Bedingungen durchgeführt. Mit Hilfe dieser Ausführungsform werden erfindungsgemäß auch wirkstoffhaltige Implantate bereitgestellt. Weitere, erfindungsgemäß unter sterilen Bedingungen bereitgestellte wirkstoffhaltige Objekte sind solche, die injizierbar sind.

Wie bereits eingangs offenbart, bezieht sich die Erfindung unter einem weiteren Gesichtspunkt auf eine Vorrichtung zur Herstellung einer festen Darreichungsform, insbesondere zur Durchführung des erfindungsgemäßen Verfahrens, mit
(a) einer Aufbauplattform, auf der die Darreichungsform gedruckt wird;
(b) mindestens einem zum Auftragen einer Anordnung von Spots einer Aufbausubstanz für die Darreichungsform auf der Aufbauplattform ausgelegten Druckkopf, wobei die Aufbausubstanz im Druckzustand fließfähig ist und nach dem Druck mindestens halbfest, vorzugsweise fest wird,
wobei die Aufbausubstanz mindestens einen pharmazeutischen Wirkstoff und/oder mindestens einen nutrazeutischen Wirkstoff und/oder mindestens einen Nahrungsergäzungsmittelwirkstoff enthält.

Bevorzugte Ausführungsformen der Vorrichtung wurden bereits im Rahmen der Offenbarung des erfindungsgemäßen Verfahrens dargelegt.

## Patentansprüche

1. Verfahren zur Herstellung einer festen Darreichungsform, umfassend die Schritte:
(i) Bereitstellen eines mindestens zum 3D-Druck der festen Darreichungsform befähigten Druckers, der
eine Aufbauplattform, auf der die Darreichungsform gedruckt wird;
einen zum Auftragen einer Anordnung von Spots einer Aufbausubstanz für die Darreichungsform auf der Aufbauplattform ausgelegten Druckkopf, wobei die Aufbausubstanz im Druckzustand, vorzugsweise im erwärmten Zustand, fließfähig ist und durch Verfestigung, vorzugsweise durch Abkühlung, mindestens halbfest wird,
(ii) Aufbringen einer Anordnung von Spots der Aufbausubstanz auf der Aufbauplattform, wobei sich die Spots überlappen oder berühren oder nicht berühren können;
(iii) mindestens Halbverfestigen, vorzugsweise Verfestigen, vorzugsweise durch Abkühlen, der im Schritt (ii) aufgebrachten Spots derart, dass sich die Aufbausubstanz mindestens halbverfestigt;
(iv) Aufbringen einer weiteren Anordnung von Spots auf die vorherige Anordnung von Spots derart, dass sich die Spots der weiteren Anordnung mit den Spots der vorherigen Anordnung überlappen; und
(v) Wiederholen der Schritte (ii) bis (iv) bis die Darreichungsform gebildet ist,
wobei die Aufbausubstanz mindestens einen pharmazeutischen Wirkstoff und/oder mindestens einen nutrazeutischen Wirkstoff und/oder mindestens einen Nahrungsergäzungsmittelwirkstoff enthält.

2. Verfahren nach Anspruch 1, wobei der Drucker mindestens einen Druckkopf aufweist, der mit einem Reservoir mit der Aufbausubstanz in Verbindung steht, so dass der mindestens eine Druckkopf zur Entnahme einer Menge der Aufbausubstanz zum Aufbringen der Aufbausubstanz in den Schritten (ii) bis (v) befähigt ist.

3. Verfahren nach Anspruch 2, wobei der Drucker mehr als einen Druckkopf , vorzugsweise 2 bis 10 oder mehr aufweist.

4. Verfahren nach Anspruch 3, wobei jeder der Druckköpfe mit einem Reservoir mit der Aufbausubstanz in Verbindung steht, so dass der jeweilige Druckkopf zur Entnahme einer Menge der Aufbausubstanz zum Aufbringen der Aufbausubstanz in den Schritten (ii) bis (v) befähigt ist.

5. Verfahren nach Anspruch 1, wobei die Aufbausubstanz als Filament vorliegt und der Druckkopf zum Abschmelzen einer Menge des Filaments ausgelegt ist, um die Anordnung von Spots der Aufbausubstanz auf der Aufbauplattform im Schritt (ii) sowie die weitere Anordnung von Spots auf der vorherigen Anordnung von Spots im Schritt (iv) aufzubringen.

6. Verfahren nach einem der Ansprüche 1 bis 5, wobei die Spots durch Aufbringen eines Volumeninkrements der Aufbausubstanz erzeugt werden und die Volumeninkremente ein Volumen von 20 pl bis 30 µl aufweisen.

7. Verfahren nach einem der vorhergehenden Ansprüche, wobei sich die im Schritt (ii) bzw. jedem weiteren Schritt (ii) gemäß Schritt (v) aufgebrachten Spots teilweise überlappen.

8. Verfahren nach einem der Ansprüche 1 bis 6, wobei sich die im Schritt (ii) bzw. jedem weiteren Schritt (ii) gemäß Schritt (v) aufgebrachten Spots vollständig überlappen.

## Claims

1. A method of preparing a solid dosage form comprising the steps of:
(i) providing a printer capable of at least 3D printing the solid dosage form, the printer comprising
a building platform on which the dosage form is printed;
a print head designed for applying an arrangement of dots of a building substance for the dosage form on the building platform, wherein the building substance is flowable, preferably through heating, when it is printed, and becomes at least semi-solid by solidification, preferably by cooling,
(ii) applying an array of dots of the building substance on the building platform, wherein the dots may overlap or contact or not contact each other;
(iii) at least semi-solidifying, preferably solidifying, preferably by cooling, the dots applied in step (ii) such that the building substance becomes at least semi-solid;
(iv) applying a further array of dots to the previous array of dots in such a manner that the dots of the further array overlap with the dots of the previous array; and
(v) repeating steps (ii) to (iv) until the dosage form is formed,
wherein the building substance comprises at least one pharmaceutically active agent and/or at least one nutraceutical active agent and/or at least one dietary supplement active agent.

2. The method of claim 1, wherein the printer comprises at least one print head connected to a reservoir containing the building substance, such that the at least one print head is capable of withdrawing an amount of the building substance for applying the building substance in steps (ii) to (v).

3. The method of claim 2, wherein the printer comprises more than one, preferably 2 to 10 or more, print head(s).

4. The method of claim 3 wherein each of the print heads is in communication with a reservoir with the building substance such that the respective print head is capable of withdrawing an amount of the building substance for applying the building substance in steps (ii) to (v).

5. The method of claim 1 wherein the building substance is in the form of a filament and the print head is designed to melt an amount of the filament for applying the array of dots of the building substance on the building platform in step (ii) and the further array of dots on the previous array of dots in step (iv).

6. The method according to any one of claims 1 to 5 wherein the dots are generated by applying a volume increment of the building substance and the volume increments have a volume of 20 pl to 30 µl.

7. The method according to any one of the preceding claims wherein the dots applied in step (ii) or each further step (ii) according to step (v) partially overlap.

8. The method according to any one of claims 1 to 6, wherein the dots applied in step (ii) or each further step (ii) according to step (v) completely overlap.

## Revendications

1. Procédé de fabrication d'une forme galénique solide, comprenant les étapes suivantes :
(i) Fournir une imprimante capable d'imprimer au moins en 3D la forme galénique solide, comprenant
une plate-forme de construction sur laquelle la forme pharmaceutique est imprimée ;
une tête d'impression conçue pour appliquer un agencement de spots d'une substance de construction pour la forme pharmaceutique sur la plate-forme de construction, la substance de construction étant fluide à l'état imprimé, de préférence à l'état chauffé, et devenant au moins semi-solide par solidification, de préférence par refroidissement,
(ii) appliquer un agencement de spots de la substance de construction sur la plate-forme de construction, les spots pouvant se chevaucher ou se toucher ou ne pas se toucher ;
(iii) au moins semi-solidifier, de préférence solidifier, de préférence par refroidissement, les spots appliqués à l'étape (ii) de telle sorte que la substance de construction se solidifie au moins à moitié ;
(iv) appliquer un autre ensemble de spots sur l'ensemble de spots précédent de telle sorte que les spots de l'ensemble supplémentaire se chevauchent avec les spots de l'ensemble précédent ; et
(v) répéter les étapes (ii) à (iv) jusqu'à ce que la forme pharmaceutique soit formée,
dans laquelle la substance de constitution contient au moins un substance pharmaceutique actif et/ou au moins une substance nutraceutique actif et/ou au moins une substance actif de complément alimentaire.

2. Procédé selon la revendication 1, dans lequel l'imprimante comprend au moins une tête d'impression en communication avec un réservoir contenant la substance d'assemblage, de sorte que ladite au moins une tête d'impression est capable de prélever une quantité de la substance d'assemblage pour appliquer la substance d'assemblage dans les étapes (ii) à (v).

3. Procédé selon la revendication 2, dans lequel l'imprimante comprend plus d'une tête d'impression, de préférence de 2 à 10 ou plus.

4. Procédé selon la revendication 3, dans lequel chacune des têtes d'impression communique avec un réservoir contenant la substance d'assemblage, de sorte que la tête d'impression respective est capable de prélever une quantité de la substance d'assemblage pour appliquer la substance d'assemblage dans les étapes (ii) à (v).

5. Procédé selon la revendication 1, dans lequel la substance de construction se présente sous la forme d'un filament et la tête d'impression est adaptée pour faire fondre une quantité du filament afin d'appliquer l'agencement de spots de la substance de construction sur la plate-forme de construction à l'étape (ii) ainsi que l'agencement supplémentaire de spots sur l'agencement de spots précédent à l'étape (iv).

6. Procédé selon l'une quelconque des revendications 1 à 5, dans lequel les spots sont créés par application d'un incrément de volume de la substance de construction et les incréments de volume ont un volume de 20 pl à 30 µl.

7. Procédé selon l'une quelconque des revendications précédentes, dans lequel les spots appliqués à l'étape (ii) ou à chaque étape (ii) ultérieure selon l'étape (v) se chevauchent partiellement.

8. Procédé selon l'une quelconque des revendications 1 à 6, dans lequel les spots appliqués à l'étape (ii) ou à chaque étape ultérieure (ii) selon l'étape (v) se chevauchent complètement.
